# EUROPEAN PATENT SPECIFICATION

(11) **EP 4 100 104 B1**
(45) Date of publication and mention of the grant of the patent: **20.08.2025**
(21) Application number: 21709208.9
(22) Date of filing: 04.02.2021
(51) Int. Cl.: A61M 60/165, A61M 60/205, A61M 60/247, A61M 60/861

(54) **INFLOW / OUTFLOW CANNULA**
ZUFLUSS-/ABFLUSSKANÜLE
CANULE D'ENTRÉE/SORTIE

(30) Priority: 04.02.2020 US 202062969824 P
(43) Date of publication of application: 14.12.2022
(73) Proprietor: W. L. Gore & Associates, Inc., Newark, DE 19711 (US)
(72) Inventor: ALSTON, JR., Steven M., Newark, Delaware 19711 (US); BRYSON, Scott M., Newark, Delaware 19711 (US); GOEPFRICH, James L., Newark, Delaware 19711 (US)
(74) Representative: HGF
(86) International application number: PCT/US2021/016532
(87) International publication number: WO 2022/169449

(56) References cited:
- US-A1- 2012 059 213

## Description

### CROSS-REFERENCE TO RELATED APPLICATION

This application claims the benefit of Provisional Application No. 62/969,824, filed February 4, 2021.

### FIELD

This disclosure relates generally to medical devices, and more specifically, to inflow or outflow cannulas that may include a tissue anchor.

### BACKGROUND

Cannulas may be able to create flow conduits within a patient. For example, cannulas may be used to create flow conduits to or from an organ such as the heart. A cannula may be used to create an inflow or outflow conduit from the heart. In certain instances, a cannula may be used to create an inflow conduit from the heart in cardiac assist applications to increase blood from the heart to assist with blood circulation in a patient. Arranging the cannula within an organ such as the heart may use an anchoring structure. US2012059213 describes a cannula assembly for directing the flow of material from an organ chamber, e.g., blood from the left chamber of the heart, and methods of placing the cannula assembly in fluidic communication with the chamber. The cannula assembly includes an elongate tubular member and a coupling assembly disposed at the distal end of elongate tubular member. The elongate tubular member includes a lumen extending from a distal opening at the distal end to a proximal opening at the proximal end. The coupling assembly includes a retaining element and a retention member configured to cooperate with each other and with the portion of the organ wall surrounding the opening in the wall to couple or anchor cannula system to the wall and to provide fluidic communication between the distal opening of the elongate tubular member and the organ chamber.

### SUMMARY

The claimed invention proposes a cannula apparatus according to claim 1 (Example 1). Optional features are mentioned in the dependent claims 2-10 :

According to another example ("Example 2"), further to the apparatus of Example 1, the graft portion is arranged on an exterior surface and an interior surface of the plurality of elongate members.

According to another example ("Example 3"), further to the apparatus of any one of Examples 1-2, the graft portion is arranged along an interior surface of the plurality of elongate members and extends along the interior surface of the conduit to a second end of the conduit.

According to another example ("Example 4"), further to the apparatus of Example 3, the graft portion extends along an exterior surface of the plurality of elongate members.

According to another example ("Example 5"), further to the apparatus of any one of Examples 1-3, the inlet portion is configured to conform to the tissue wall and lessen tissue erosion thereof.

According to another example ("Example 6"), further to the apparatus of any one of Examples 1-5, the exterior surface of the conduit is configured to lessen thrombus formation.

According to another example ("Example 7"), further to the apparatus of any one of Examples 1-6, the inlet portion includes a funnel shape in an initial configuration and is configured to flatten and deploy against the tissue wall.

According to another example ("Example 8"), further to the apparatus of any one of Examples 1-7, the apparatus also includes a constraining ring arranged about the plurality of elongate members to maintain the inlet portion in a substantially cylindrical configuration and allow release of the inlet portion to flatten and deploy against the tissue wall.

According to another example ("Example 9"), further to the apparatus of Example 8, the constraining ring is configured to slide away from the first end of the conduit in response to contact with an epicardial surface of the heart to allow release of the inlet portion.

According to another example ("Example 10"), further to the apparatus of any one of Examples 1-9, the apparatus also includes a plurality of ring structures arranged along the exterior surface of an outflow portion of the conduit.

According to another example ("Example 11"), further to the apparatus of any one of Examples 1-10, the inlet portion is configured to deploy against the tissue wall in response to fluid flow pressure with a heart chamber.
While multiple examples of the claimed invention are disclosed, still other embodiments will become apparent to those skilled in the art from the following detailed description, which shows and describes illustrative examples. Accordingly, the drawings and detailed description are to be regarded as illustrative in nature rather than restrictive in nature. The scope of the claimed invention is only limited by the claims.

### BRIEF DESCRIPTION OF THE DRAWINGS

The accompanying drawings are included to provide a further understanding of the disclosure and are incorporated in and constitute a part of this specification, illustrate embodiments, and together with the description serve to explain the principles of the disclosure.
FIG. 1 is an illustration of an example anchoring structure, which is not part of the claimed invention but present for illustration purpose only,
FIGS. 2A-D are example cut-patterns for inlet portions, in accordance with various aspects of the present disclosure;
FIG. 3 is an illustration of an example cannula, in accordance with various aspects of the present disclosure;
FIG. 4 is an illustration of an example inlet portion arranged within heart tissue, in accordance with various aspects of the present disclosure;
FIG. 5 is an example system that includes an inflow cannula, an outflow, cannula, and a pump, in accordance with various aspects of the present disclosure;
FIG.6A is a side view of an example inlet portion and a constraining ring in a delivery configuration; which is not part of the claimed invention but present for illustration purpose only,
FIG. 6B is a top view of the inlet portion and the constraining ring, shown in FIG. 6A, in the delivery configuration, which is not part of the claimed invention but present for illustration purpose only,
FIG. 6C is a side vide of the inlet portion and the constraining ring, shown in FIGS. 6A-B, in a second configuration, which is not part of the claimed invention but present for illustration purpose only,
FIGS. 7A-C are illustrations of cut-patterns for inlet portions, in accordance with various aspects of the present disclosure;
FIG. 8A is a side view of an example cannula and inlet portion, which is not part of the claimed invention but present for illustration purpose only,
FIG. 8B is a top view of the inlet portion, shown in FIG. 8A, which is not part of the claimed invention but present for illustration purpose only,
FIG. 9A is a side view of another example cannula and inlet portion, in accordance with various aspects of the present disclosure;
FIG. 9B is a top view of the inlet portion, shown in FIG. 9A, in accordance with various aspects of the present disclosure;
FIG. 10A is a side view of another example cannula and inlet portion, which is not part of the claimed invention but present for illustration purpose only,
FIG. 10B is a top view of the inlet portion, shown in FIG. 10A, which is not part of the claimed invention but present for illustration purpose only,
FIG. 11 is a perspective view of an example adjustable cannula portion, in accordance with various aspects of the present disclosure;
FIG. 12A is a side view of an example adjustable cannula portion and inlet portion in a first configuration, in accordance with the claimed invention, **and**
FIG. 12B is a side view of the adjustable cannula portion and inlet portion, shown in FIG. 12A, in a second configuration, in accordance with the claimed invention.

### DETAILED DESCRIPTION

### Definitions and Terminology

This disclosure is not meant to be read in a restrictive manner. For example, the terminology used in the application should be read broadly in the context of the meaning those in the field would attribute such terminology.

With respect to terminology of inexactitude, the terms "about" and "approximately" may be used, interchangeably, to refer to a measurement that includes the stated measurement and that also includes any measurements that are reasonably close to the stated measurement. Measurements that are reasonably close to the stated measurement deviate from the stated measurement by a reasonably small amount as understood and readily ascertained by individuals having ordinary skill in the relevant arts. Such deviations may be attributable to measurement error, differences in measurement and/or manufacturing equipment calibration, human error in reading and/or setting measurements, minor adjustments made to optimize performance and/or structural parameters in view of differences in measurements associated with other components, particular implementation scenarios, imprecise adjustment and/or manipulation of objects by a person or machine, and/or the like, for example. In the event it is determined that individuals having ordinary skill in the relevant arts would not readily ascertain values for such reasonably small differences, the terms "about" and "approximately" can be understood to mean plus or minus 10% of the stated value.

### Description of Various Embodiments

Persons skilled in the art will readily appreciate that various aspects of the present disclosure can be realized by any number of methods and apparatuses configured to perform the intended functions. It should also be noted that the accompanying drawing figures referred to herein are not necessarily drawn to scale, but may be exaggerated to illustrate various aspects of the present disclosure, and in that regard, the drawing figures should not be construed as limiting.

Various aspects of the present disclosure are directed to apparatuses, systems, and methods that may be used in improving or assisting the cardiac function of the heart. The disclosed to apparatuses, systems, and methods generally include an anchoring structure or inlet portion of a cannula that may be arranged within a patient's heart. The disclosed anchoring structure or inlet portion of a cannula may be used with a pump system for increasing blood flow in a patient.

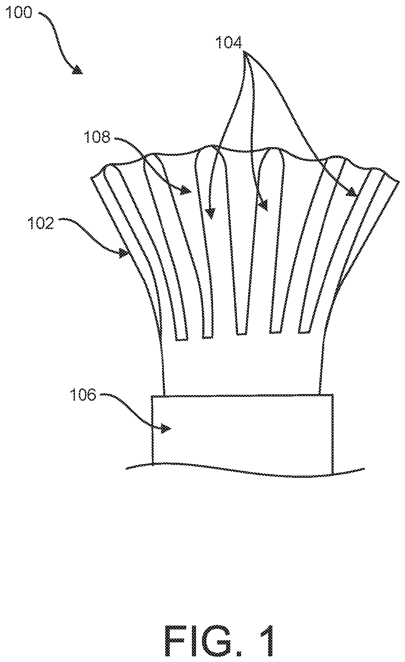

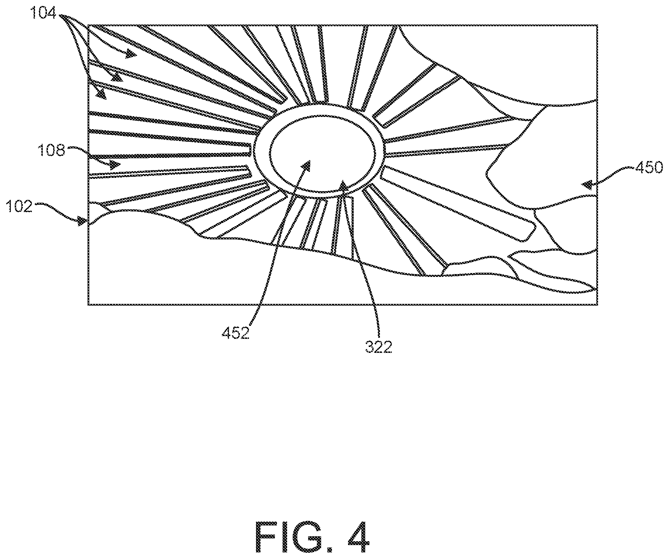

FIG. 1 is an illustration of an example anchoring structure 100, in accordance with various aspects of the present disclosure. The anchoring structure 100 may be used for an inflow or outflow cannula. The anchoring structure 100 includes an inlet portion 102 including a plurality of elongate members 104 arranged about a circumference of the inlet portion 102. The plurality of elongate members 104 configured to deploy against a tissue wall (e.g., as shown in FIG. 4).

The anchoring structure 100 also includes an outflow portion 106 arranged distal of the inlet portion 102 and including an exterior surface and an interior surface. A graft portion 108 may cover and be arranged between the plurality of elongate members 104 as is shown in FIG. 1. In addition, the graft portion 108 extends along an interior surface of the outflow portion 106. The graft portion 108 being arranged along an interior portion of the inlet portion 102 and the outlet portion 108 may lessen thrombus formation along the interior surface. The interior surface is the luminal surface for blood flow when the anchoring structure 100 is arranged within the patient. The graft portion 108 extending along the interior surface of the inflow portion 102 and the outflow portion 106 may lessen surface disturbances along the blood flow surface of the anchoring structure 100.

In certain instances, the graft portion 108 may also extend about a distal end of the inlet portion 102 and cover an exterior surface of the inlet portion 102. In other instances, a second graft portion or layer may extend to cover the exterior surface of the inlet portion 102. The graft portion 108 may extend between and within the spaces of the plurality of elongate members 104. In certain instances, the graft portion 108 may be configured to stretch and recover in response to forces acting on the graft portion 108. The stretching may facilitate conformability. For further discussion regarding the material properties of the graft portion 108, reference may be made to U.S. Patent No. 4,877,661 ("House, et al.").

As shown, the inlet portion 102 may include a funnel shape in an initial configuration. The plurality of elongate members 104 may be shape set in the funnel shape in certain instances. The inlet portion 102 may be configured to flatten and deploy against the tissue wall. In certain instances, the inlet portion 102 is configured to deploy against the tissue wall in response to fluid flow pressure with a heart chamber.

As discussed in further detail below, the anchoring structure 100 may be arranged with an outflow or inflow cannula and coupled to a pump. The anchoring structure 100 may contact the tissue wall of a patient's heart and stabilize the cannula. The anchoring structure 100 is configured to contact and conform to the tissue wall and provide an inlet for blood flow out of the heart. The anchoring structure 100 may lessen stasis and thrombus formation by maintaining contact against the tissue wall as discussed in further detail below. The plurality of elongate members 104 may stabilize the anchoring structure 100 and the graft portion 108 may cover the entirety of the plurality of elongate members 104 to lessen tissue erosion.

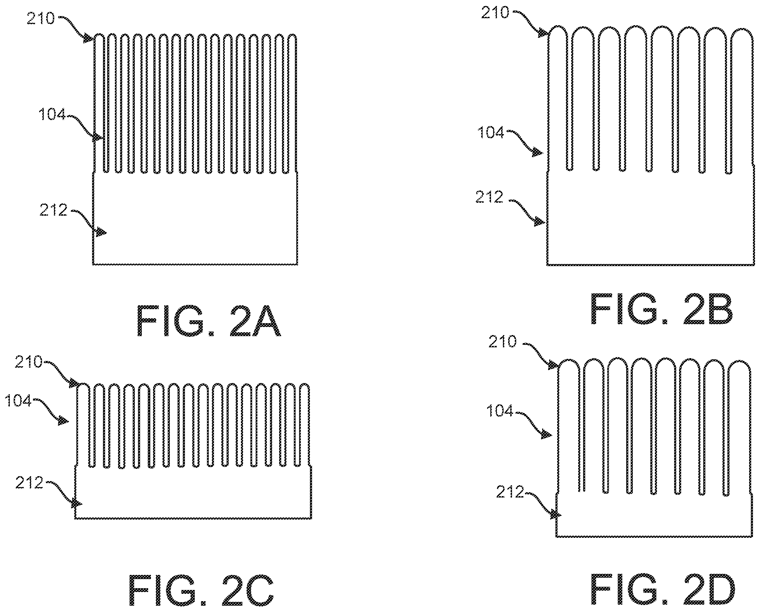

FIGS. 2A-D are example cut-patterns for inlet portions, in accordance with various aspects of the present disclosure. A plurality of elongate members 104 may be formed from any one of or a combination of the cut-patterns shown in FIGS. 2A-D. The plurality of elongate members 104 may be formed from a flat-sheet or tube of Nitinol (NiTi). In certain instances, the plurality of elongate members 104 may be formed of a polymer material or a different biocompatible metal such as stainless steel. The plurality of elongate members 104 may include an atraumatic distal end 210. The distal end 210 of each of the plurality of elongate members 104 may be curved.

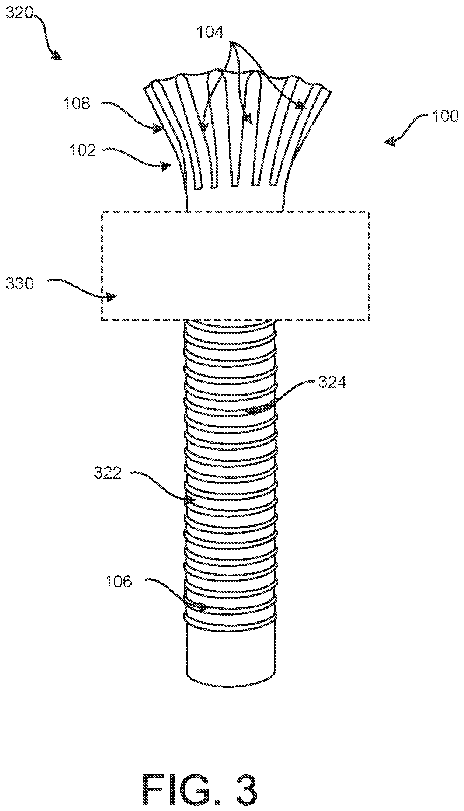

In addition, the patterns that form the plurality of elongate members 104 may be formed into an inlet portion or anchoring structure as shown in FIGS. 1 and 3, for example, the number of plurality of elongate members 104 may vary as shown in FIGS. 2A-D. For example, the pattern shown in FIG. 2A includes 16 elongate members 104 and the pattern shown in FIG. 2B includes 8 elongate members 104. Patterns may be formed to include 6, 8, 10, 12, 14, 16, 18, 20, 22, 24, 26, or any number therebetween number of elongate members 104.

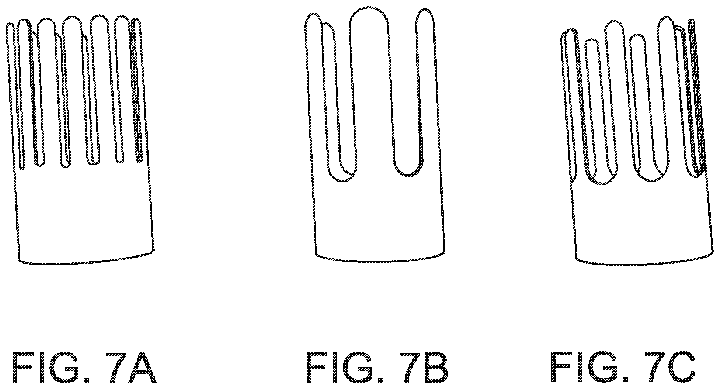

In addition and as shown, the patterns include a shoulder portion 212 that the plurality of elongate members 104 extend from. The height or length may be varied as shown in comparing the patterns shown in FIGS. 2A-D. In addition, the spacing between the elongate members 104 may be varied as shown in comparing the patterns shown in FIGS. 2A-D. The thickness of the tube or flat sheet used to form the elongate members 104 may be between .2 and .5 mm thick. In addition, the patterns may be formed into a funnel structure, as shown in FIGS. 1 and 3, to include approximately a 10 mm to 20 mm outer diameter. A three dimensional form of the patterns is shown, for example, in FIGS. 7A-C.

FIG. 3 is an illustration of an example cannula 320, in accordance with various aspects of the present disclosure. The cannula 320 may be used as an inflow or outflow cannula apparatus. The cannula 320 may include a conduit 322 having an exterior surface and an interior surface. The cannula 320 may also include an inlet portion 102 arranged at a first end of the conduit 322. The inlet portion 102 may include a plurality of elongate members 104 arranged about a circumference of the inlet portion 102 that are configured to deploy against a tissue wall.

The cannula 320 may also include a graft portion 108 covering and arranged between the plurality of elongate members 104 and extending along the interior surface of the conduit 322. In certain instances, the graft portion 108 is arranged on an exterior surface and an interior surface of the plurality of elongate members 104. In addition, the graft portion 108 may be arranged along an interior surface of the plurality of elongate members 104 and extend along the interior surface of the conduit 322 to a second end 106 (or outflow portion) of the conduit. The graft portion 108 may also extend along an exterior surface of the plurality of elongate members 104.

The inlet portion 102 may contact the tissue wall of a patient's heart and stabilize the cannula 320. The inlet portion 102, by way of the elongate members 104 and the graft portion 108, may be configured to contact and conform to the tissue wall and provide an inlet for blood flow out of the heart. The inlet portion 102 may lessen stasis and thrombus formation by maintaining contact against the tissue wall as opposed to extending into the heart. The plurality of elongate members 104 may stabilize the anchoring structure 100 and the graft portion 108 may cover the entirety of the plurality of elongate members 104 to lessen tissue erosion.

As shown, the inlet portion 102 includes a funnel shape in an initial configuration and is configured to flatten and deploy against a tissue wall (e.g., as shown in FIG. 4). The inlet portion 102 is configured to deploy against the tissue wall in response to fluid flow pressure with a heart chamber. In addition and as noted above, the graft portion 108 may be arranged along an interior surface of the plurality of elongate members 104 and extend along the interior surface of the conduit 322. The graft portion 108 being arranged along an interior portion of the inlet portion 102 and the conduit 322 may lessen thrombus formation along the interior surface. The interior surface is the luminal surface for blood flow within the cannula 320. The graft portion 108 extending along the interior surface of the inflow portion 102 and the conduit 322 may lessen surface disturbances along the blood flow surface of the cannula 320.

A plurality of ring structures 324 arranged may be along the exterior surface of an outflow portion 106 of the conduit 322. The plurality of ring structures 324 may be a biocompatible non-metallic material such as densified ePTFE. For further discussion to densified ePTFE, reference may be made to U.S. Patent Nos. 5,843,171 ("Campbell et al.") and 5,747,128 ("Campbell et al."). The plurality of ring structures 324 may facilitate kink resistance and hoop strength of the outflow portion 106 of the conduit 322.

In certain instances, the graft portion 108 may include a coating that is configured to minimize a thrombogenic response that may occur due to exposure of the graft portion 108 to blood contact. In certain instances, the coating may be a heparin coating. The heparin coating is utilized to bind heparin molecules to the graft portion 108. For further discussion regarding a heparin coated graft portion 108, reference may be made to U.S. Patent No. 6,461,665 ("Scholander") for the specific teachings of antithrombogenic activity of surface immobilized heparin. In certain instances, the heparin coating may be CARMEDA^{®} BioActive Surface (CBAS^{®} Heparin Surface).

In certain instances, the heparin coating may be applied to the graft portion 108 in one or more layers. The chemical constituents of the covering material in each layer can be the same or different. In some instances, the covering material is cross-linked to itself or other covering materials in other layers. The cross-linking bonds can be covalent or ionic. The heparin covering may form at least one layer on at least a portion of the graft portion 108 and may cross-link to itself or other layers of the covering. The cross-linking can be covalent, ionic, or both. For reference regarding the application of layers of heparin to the graft portion 108, reference may be made to U.S. Patent No. 9,399,085 (Cleek et al.).

In some instances, the graft portion 108 is configured to induce rapid tissue ingrowth therein on at least a portion of an exterior (tissue contacting) surface. In these instances, the configuration or material properties of the graft portion 108 covering the exterior surface of the inlet portion 102 may be different than properties of the interior graft portion 108. The graft portion 108 may be different layers of the same graft portion 108 or the exterior may include a different second graft portion. For ingrowth, the graft portion 108 can have a microporous structure that provides a tissue ingrowth scaffold for durable occlusion and supplemental anchoring strength of plurality of elongate members 104.

In certain instances, the cannula 320 may include a coupling section 330. The coupling section 330 may be used to seal and secure the cannula 320 with an opening created in the tissue. For example, an opening may be created in the heart (ventricle or atrium) when the cannula 320 is arranged therein. The inlet portion 102, as noted above, is arranged on an interior wall of the heart tissue (as also shown in FIG. 4). The coupling section 330 may seal the opening and hold the cannula 320 in place. In certain instances, sutures may be arranged through the coupling section 330 to secure the coupling section 330 to the heart tissue.

FIG. 4 is an illustration of an example inlet portion 102 arranged within heart tissue 450, in accordance with various aspects of the present disclosure. The inlet portion 102 includes a plurality of elongate members 104 with a graft portion 108 attached to an exterior surface (contacting the tissue wall 450) and an interior surface (shown in FIG. 4). As discussed in detail above, the graft portion 108 covers spaces or gaps between the elongate members 104.

As shown in FIG. 4, the inlet portion 102 contacts the tissue wall 450 in a deployed configuration. The inlet portion 102, by way of the elongate members 104 and the graft portion 108, may be configured to contact and conform to the tissue wall and provide an inlet for blood flow out of the heart. The inlet portion 102 may lessen stasis and thrombus formation by maintaining contact against the tissue wall as opposed to extending into the heart. The plurality of elongate members 104 may stabilize the anchoring structure 100 and the graft portion 108 may cover the entirety of the plurality of elongate members 104 to lessen tissue erosion. The tissue wall 450 may be within the heart (e.g., left ventricle, left atrium, right ventricle, right atrium) in certain instances. In addition, the anchoring structure 100 may be arranged within an appendage of the heart (e.g., left atrial appendage).

In addition and as is shown, the graft portion 108 may be arranged along an interior surface of the plurality of elongate members 104 and extend along an interior surface 452 of a conduit 322 to a second end (or outflow portion) of the conduit.

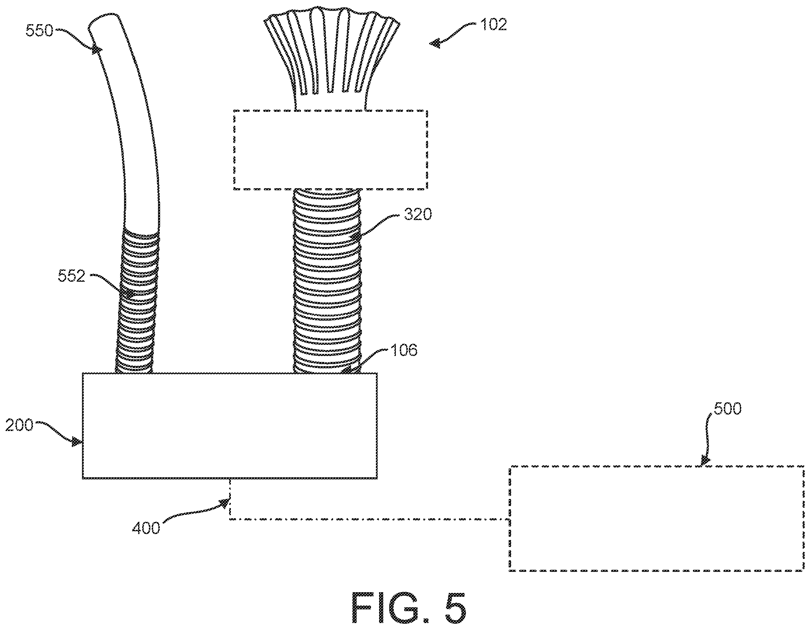

FIG. 5 is an example system that includes an inflow cannula 320, an outflow cannula 550, and a pump 200, in accordance with various aspects of the present disclosure. As described in detail above, the inflow cannula 320 may include an inlet portion 102, and may be coupled to a pump 200 at an outflow portion 106.

The pump 200 may generally be any pump 200 that is configured to drive or otherwise cause blood to flow across the pump 200 through the cannula 320 from the inlet portion 102 to and through the outflow cannula 550. The outflow cannula 550, as shown, may include a plurality of ring structures 552 to facilitate kink resistance and hoop strength as noted above with reference to the ring structures 324 on the inflow cannula 320. The pump mechanism (also referred to herein as a pump drive) of the pump 200 may be, for example, a centrifugal-action pump, an axial-action pump, a positive displacement pump, a pulsatile pump, or other similar device such as a worm-style drive mechanism, or impeller. The pump 200 can be operated to draw blood from the left ventricle (or other heart chamber) and across the pump 200.

In certain instances, the system further includes a driveline 400. The driveline 400 is a cable assembly that operates to electrically couple a controller 500 located external to the patient's anatomy with the pump 200 or the driveline 400 can be a rotating driveshaft. The driveline 400 may be routed through the patient's vasculature and then out through the skin to where it is coupled with the controller 500 or to a subcutaneously implanted controller 500. The controller 500 is a module that is configured to control the operation of the pump 200. The controller 500 may include a battery to control operation of the pump 200. In certain instances, the controller 500 may be integrated into the pump 200 such that the system may be configured to operate without the need for the driveline 400, or the driveline 400 need not extend extracorporeally.

In addition, an extracorporeal control system may be configured to both control the operation of the pump, and to power the pump wirelessly (e.g., through a transcutaneous energy transmission system). In some examples, transcutaneous energy transmission may be accomplished through known means of transcutaneous energy transmission, such as those described in U.S. Patent No. 6,400,991. Such a configuration eliminates the need to route the driveline 400 through the vasculature and out through a percutaneous access site, which can help minimize a risk for infection. In some examples, the pump 200 may include an "antenna" (or internal coil) that is configured for transcutaneous energy transfer ("TET"). In some examples, an extracorporeal TET component maybe worn around the torso similar to a standard heart rate monitor, and additionally coupled to a power source (wall unit or high capacity battery) such that the extracorporeal TET component is operable to transmit energy transcutaneously to the antenna.

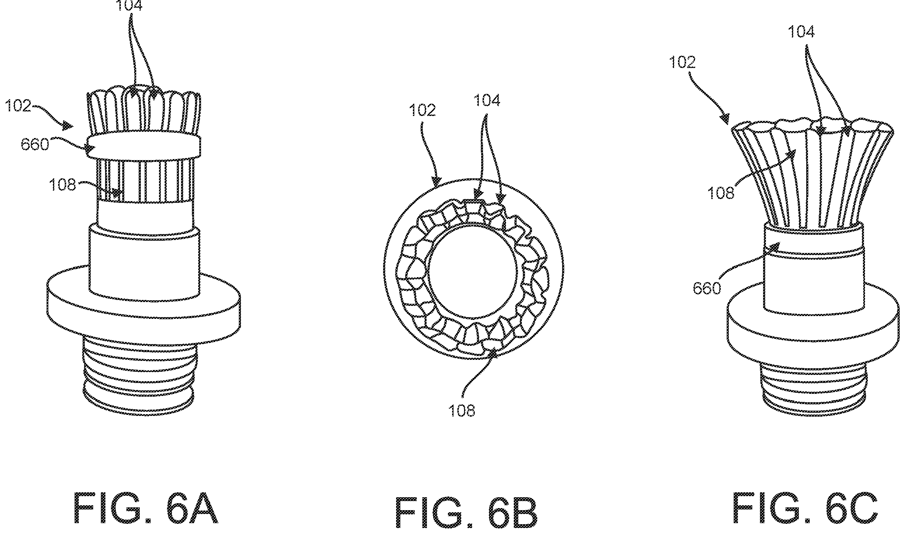

FIG.6A is a side view of an example inlet portion 102 and a constraining ring 650 in a delivery configuration; in accordance with various aspects of the present disclosure. As shown, the inlet portion 102 (which includes a plurality of elongate members 104 and a graft portion 108) is constrained and maintained in a substantially cylindrical configuration/shape in the delivery configuration by the constraining ring 650. FIG. 6B is a top view of the inlet portion 102 and the constraining ring 660, shown in FIG. 6A, in the delivery configuration, in accordance with various aspects of the present disclosure;

The constraining ring 650 may be configured to slide away from the inlet end of the conduit 322 (*e.g.*, the inlet end) in response to contact with an epicardial surface of the heart to allow release of the inlet portion. FIG. 6C is a side view of the inlet portion 102 and the constraining ring 650, shown in FIGS. 6A-B, in a second configuration where the constraining ring 650 has been slid to release the inlet portion 102. In certain instances, the inlet portion 102 (e.g., the anchoring structure) is arranged within a patient's heart in the delivery configuration shown in FIG. 6A. The inlet portion 102 (*e.g.*, the anchoring structure) is moved within the heart, and the inlet portion 102 is allowed to expand from the delivery configuration to deploy against the tissue wall. An opening or hole in the heart (or other tissue) may be formed prior to moving the inlet portion 102 within the heart. The constraining ring 650, in certain instances, may contact an exterior surface of the heart or organ and slide away from an end of the inlet portion 102 to allow release of the inlet portion 102 to flatten and deploy against the tissue wall (e.g., as shown in FIG. 4).

Moving the inlet portion 102 includes forcing the constraining ring 650 against an epicardial surface of the heart to allow release of the inlet portion 102, in certain instances. The opening created in the heart tissue may be smaller than an outer diameter of the constraining ring 650 to ensure that the constraining ring 650 does not enter the heart. In certain instances, an actuator or line may be coupled to the constraining ring 650 such that a physician may manually actuate the constraining ring 650.

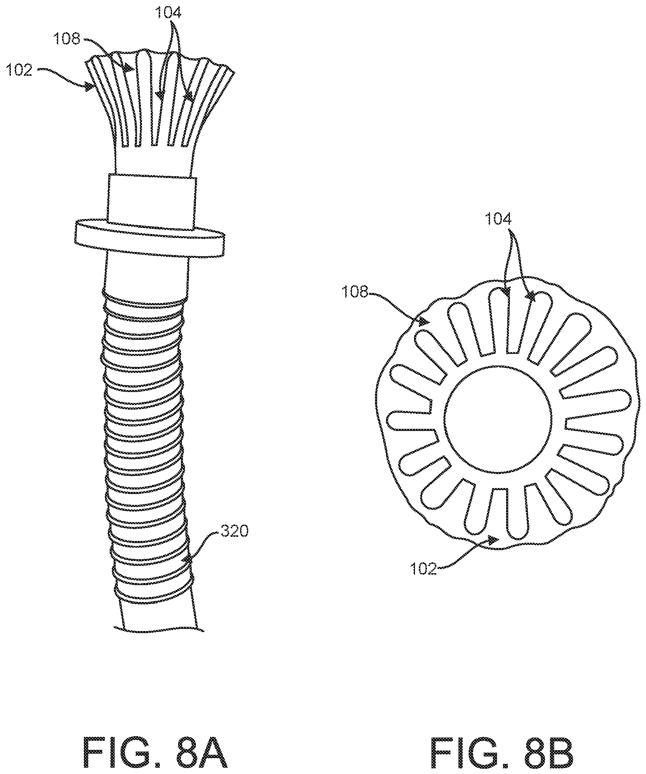

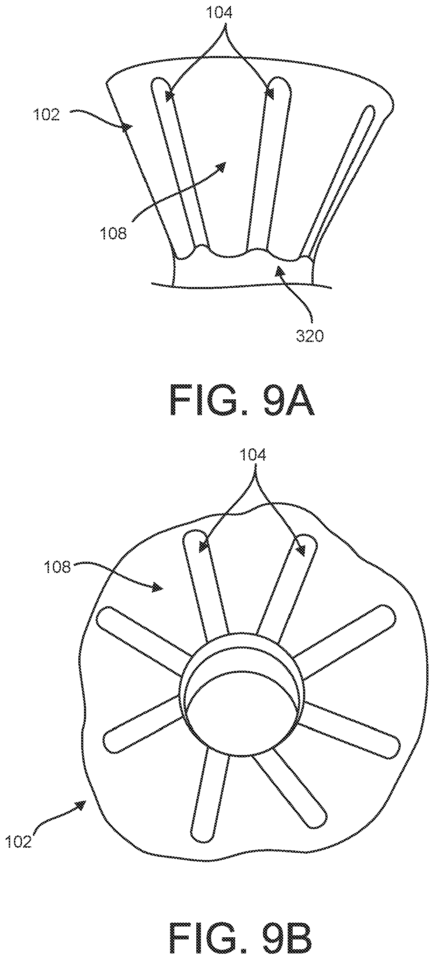

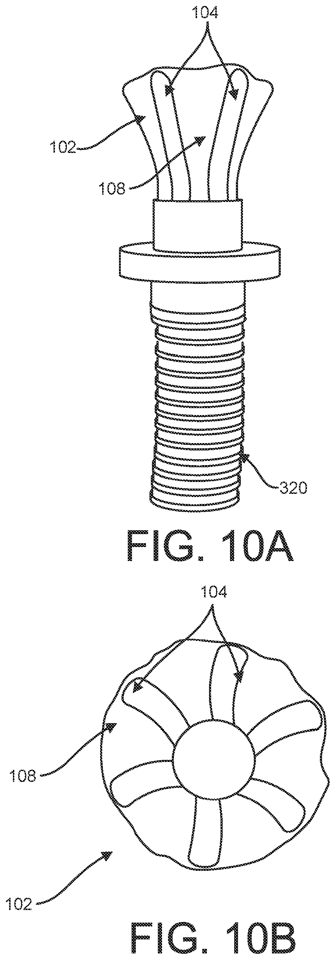

FIGS. 8-10 show various configurations of a cannula 320 and inlet portion 102 consistent with the various aspects of the present disclosure. The aspects of the inlet portions 102 differing between FIGS. 8-10 generally relates to a number of elongate members as described in detail above.

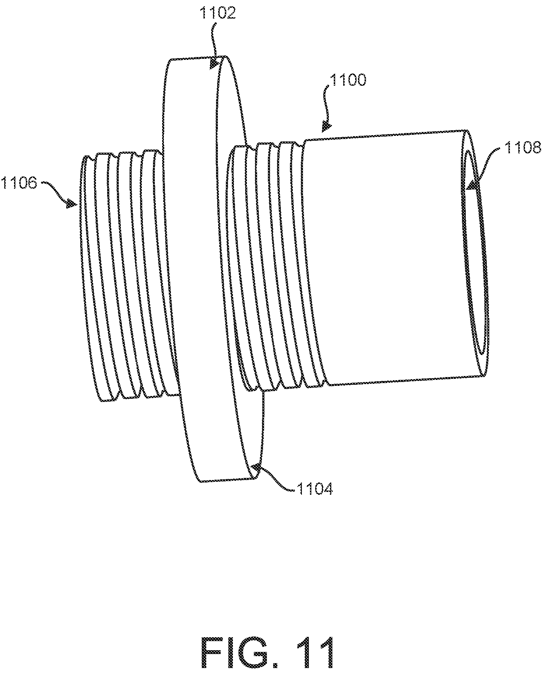

FIG. 11 is a perspective view of an example adjustable cannula portion 1100, in accordance with various aspects of the present disclosure. The adjustable cannula portion 1100 may form a portion of a cannula 320 (e.g., an inflow or outflow) as discussed in detail above. The adjustable cannula portion 1100 may include a stop 1102 that may move along a length of the adjustable cannula portion 1100. In certain instances, at least a portion of an outer surface of the adjustable cannula portion 1100 may be a threaded surface 1104. The stop 1102 may travel along a length of the threaded surface 1104. The stop 1102 may be positioned at a desired location along the threaded surface 1104 to adjust an amount of the adjustable cannula portion 1100 that is arranged within a patient's heart.

In certain instances, an inlet portion 102 (as discussed in detail above) may be arranged at a first end 1106 of the adjustable cannula portion 1100. A conduit 322 (as discussed in detail above) may form a portion of the adjustable cannula portion 1100 a second end 1108 of the adjustable cannula portion 1100.

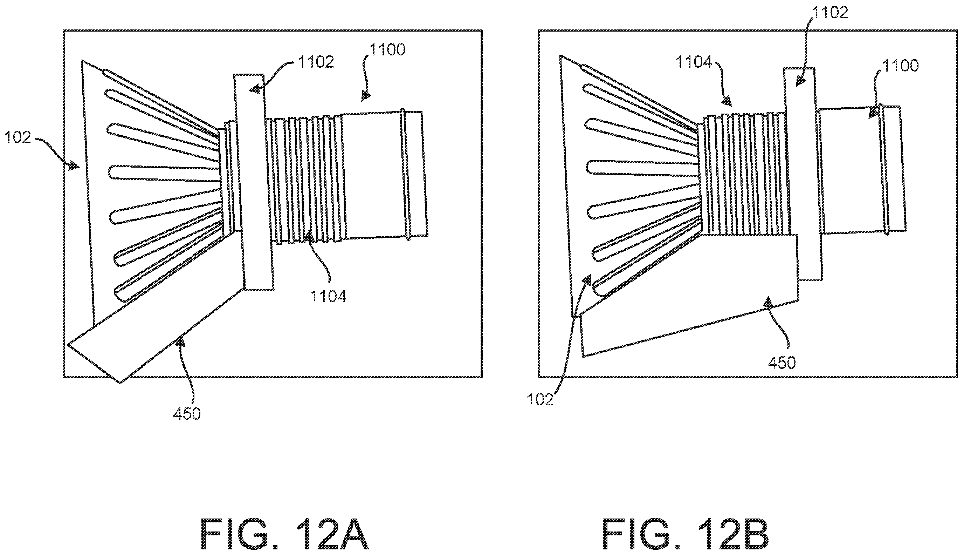

FIG. 12A is a side view of an example adjustable cannula portion 1100 and inlet portion 102 in a first configuration, in accordance with the claimed invention. FIG. 12B is a side view of the adjustable cannula portion 1100 and inlet portion 102, shown in FIG. 12A, in a second configuration. As shown in comparing FIG. 12A and FIG. 12B, a stop 1102 is arranged along a length of the adjustable cannula portion 1100 along a threaded surface 1104.

In certain instances, the closer the stop 1102 is arranged to the inlet portion 102, the less the inlet portion 102 is arranged within the heart. For example and as shown in FIG. 12A and FIG. 12B, a tissue wall 450 is arranged between the inlet portion 102 and the stop 1102. The closer the stop 1102 is arranged to the inlet portion 102, the less amount of tissue wall 450 is arranged between the inlet portion 102 and the stop 1102. The slope of the tissue wall 450 may influence the desired location of the stop 1102 along the threaded surface 1104. The adjustable cannula portion 1100 may facilitate or allow for a desired position of the inlet portion 102 such that the cannula does not substantially protrude within the heart. In certain instances, the customizable length may facilitate lessening of stasis and thrombus formation by maintaining contact of the inlet portion 102 against the tissue wall as opposed to extending into the heart. The stop 1102 is configured to travel along a length of the threaded surface 1104 to adjust a position of the inlet portion 102 relative to the tissue wall 450. The threaded surface 1104 or other portion of the adjustable cannula portion 1100 and/or the stop 1102 may include a locking feature (*e.g.*, tabs, frictional engagement members) to hold and/or lock the stop 1102in place along the threaded surface 1104.

A biocompatible material for the graft components, discussed herein, may be used. In certain instances, the graft may include a fluoropolymer, such as a polytetrafluoroethylene (PTFE) polymer or an expanded polytetrafluoroethylene (ePTFE) polymer. In some instances, the graft may be formed of, such as, but not limited to, a polyester, a silicone, a urethane, a polyethylene terephthalate, or another biocompatible polymer, or combinations thereof. In some instances, bioresorbable or bioabsorbable materials may be used, for example a bioresorbable or bioabsorbable polymer. In some instances, the graft can include Dacron, polyolefins, carboxy methylcellulose fabrics, polyurethanes, or other woven, non-woven, or film elastomers.

In addition, nitinol (NiTi) may be used as the material of the frame or stent (and any of the frames discussed herein), but other materials such as, but not limited to, stainless steel, L605 steel, polymers, MP35N steel, polymeric materials, Pyhnox, Elgiloy, or any other appropriate biocompatible material, and combinations thereof, can be used as the material of the frame. The super-elastic properties and softness of NiTi may enhance the conformability of the stent. In addition, NiTi can be shape-set into a desired shape. That is, NiTi can be shape-set so that the frame tends to self-expand into a desired shape when the frame is unconstrained, such as when the frame is deployed out from a delivery system.

In certain instances, the coating, as discussed in detail above, may include bio-active agents in addition to heparin or alternatively to heparin. The agents can include, but are not limited to, vasodilator, anti-coagulants, anti-platelet, antithrombogenic agents.

The invention of this application has been described above both generically and with regard to specific embodiments. It will be apparent to those skilled in the art that various modifications and variations can be made in the embodiments without departing from the scope of the claimed invention as defined by the appended claims 1-10. Thus, it is intended that the embodiments cover the modifications and variations of this invention provided they come within the scope of the appended claims.

## Claims

1. An inflow or outflow cannula apparatus (320), the apparatus comprising:
a conduit (322) having an exterior surface and an interior surface;
an inlet portion (102) arranged at a first end of the conduit including a plurality of elongate members (104) arranged about a circumference of the inlet portion (102) configured to deploy against a tissue wall;
and
a graft portion (108) covering and arranged between the plurality of elongate members (104) and extending along the interior surface of the conduit (322), the inflow or outflow cannula apparatus being **characterized in that** the conduit includes an adjustable cannula portion (1100) having a threaded surface (1104) and a stop (1102), and the stop is configured to travel along a length of the threaded surface (1104) to adjust a position of the inlet portion (102) relative to the tissue wall.

2. The apparatus of claim 1, wherein the graft portion (108) is arranged on an exterior surface and an interior surface of the plurality of elongate members (104).

3. The apparatus of any one of claims 1-2, wherein the graft portion (108) is arranged along an interior surface of the plurality of elongate members (104) and extends along the interior surface of the conduit (322) to a second end of the conduit; and optionally
wherein the graft portion (108) extends along an exterior surface of the plurality of elongate members (104).

4. The apparatus of any one of claims 1-3, wherein the inlet portion (102) is configured to conform to the tissue wall and lessen tissue erosion thereof.

5. The apparatus of any one of claims 1-4, wherein the exterior surface of the conduit (322) is configured to lessen thrombus formation.

6. The apparatus of any one of claims 1-5, wherein the inlet portion (102) includes a funnel shape in an initial configuration and is configured to flatten and deploy against the tissue wall.

7. The apparatus of any one of claims 1-6, further comprising a constraining ring (650) arranged about the plurality of elongate members (104) to maintain the inlet portion (102) in a substantially cylindrical configuration and allow release of the inlet portion (102) to flatten and deploy against the tissue wall; and optionally
wherein the constraining ring (650) is configured to slide away from the first end of the conduit (322) in response to contact with an epicardial surface of the heart to allow release of the inlet portion (102).

8. The apparatus of any one of claims 1-7, further comprising a plurality of ring structures (324) arranged along the exterior surface of an outflow portion of the conduit (322).

9. The apparatus of any one of claims 1-8, wherein the inlet portion (102) is configured to deploy against the tissue wall in response to fluid flow pressure with a heart chamber.

10. The apparatus of any one of claims 1-9, wherein the stop (1102) further includes a locking feature to hold and/or lock the stop (1102) in place along the threaded surface (1104).

## Patentansprüche

1. Zufluss- oder Abflusskanülenvorrichtung (320), wobei die Vorrichtung umfasst:
eine Leitung (322), die eine Außenfläche und eine Innenfläche aufweist;
einen Einlassabschnitt (102), der an einem ersten Ende der Leitung angeordnet ist und eine Vielzahl von länglichen Elementen (104) beinhaltet, die um einen Umfang des Einlassabschnitts (102) angeordnet und dazu konfiguriert sind, sich gegen eine Gewebewand zu entfalten; und
einen Graftabschnitt (108), der die Vielzahl von länglichen Elementen (104) abdeckt und zwischen diesen angeordnet ist und sich entlang der Innenfläche der Leitung (322) erstreckt, wobei die Zufluss- oder Abflusskanülenvorrichtung **dadurch gekennzeichnet ist, dass** die Leitung einen einstellbaren Kanülenabschnitt (1100) mit einer Gewindefläche (1104) und einem Anschlag (1102) beinhaltet und der Anschlag dazu konfiguriert ist, sich entlang einer Länge der Gewindefläche (1104) zu bewegen, um eine Position des Einlassabschnitts (102) relativ zu der Gewebewand einzustellen.

2. Vorrichtung nach Anspruch 1, wobei der Graftabschnitt (108) an einer Außenfläche und einer Innenfläche der Vielzahl von länglichen Elementen (104) angeordnet ist.

3. Vorrichtung nach einem der Ansprüche 1-2, wobei der Graftabschnitt (108) entlang einer Innenfläche der Vielzahl von länglichen Elementen (104) angeordnet ist und sich entlang der Innenfläche der Leitung (322) zu einem zweiten Ende der Leitung erstreckt; und optional,
wobei sich der Graftabschnitt (108) entlang einer Außenfläche der Vielzahl von länglichen Elementen (104) erstreckt.

4. Vorrichtung nach einem der Ansprüche 1-3, wobei der Einlassabschnitt (102) dazu konfiguriert ist, sich an die Gewebewand anzupassen und die Gewebeerosion davon zu verringern.

5. Vorrichtung nach einem der Ansprüche 1-4, wobei die Außenfläche der Leitung (322) dazu konfiguriert ist, Thrombusbildung zu verringern.

6. Vorrichtung nach einem der Ansprüche 1-5, wobei der Einlassabschnitt (102) eine Trichterform in einer anfänglichen Konfiguration beinhaltet und dazu konfiguriert ist, sich abzuflachen und gegen die Gewebewand zu entfalten.

7. Vorrichtung nach einem der Ansprüche 1-6, ferner umfassend einen Beschränkungsring (650), der um die Vielzahl von länglichen Elementen (104) angeordnet ist, um den Einlassabschnitt (102) in einer im Wesentlichen zylindrischen Konfiguration zu halten und die Freigabe des Einlassabschnitts (102) zu ermöglichen, sodass er sich abflacht und gegen die Gewebewand entfaltet; und optional,
wobei der Beschränkungsring (650) dazu konfiguriert ist, als Reaktion auf Kontakt mit einer epikardialen Oberfläche des Herzens von dem ersten Ende der Leitung (322) weg zu gleiten, um die Freigabe des Einlassabschnitts (102) zu ermöglichen.

8. Vorrichtung nach einem der Ansprüche 1-7, ferner umfassend eine Vielzahl von Ringstrukturen (324), die entlang der Außenfläche eines Abflussabschnitts der Leitung (322) angeordnet sind.

9. Vorrichtung nach einem der Ansprüche 1-8, wobei der Einlassabschnitt (102) dazu konfiguriert ist, sich als Reaktion auf Fluidströmungsdruck innerhalb einer Herzkammer gegen die Gewebewand zu entfalten.

10. Vorrichtung nach einem der Ansprüche 1-9, wobei der Anschlag (1102) ferner ein Verriegelungsmerkmal beinhaltet, um den Anschlag (1102) entlang der Gewindefläche (1104) in Position zu halten und/oder zu verriegeln.

## Revendications

1. Appareil à canule d'entrée ou de sortie (320), l'appareil comprenant :
un conduit (322) comportant une surface extérieure et une surface intérieure ;
une partie entrée (102) disposée au niveau d'une première extrémité du conduit comprenant une pluralité d'éléments allongés (104) disposés autour de la circonférence de la partie entrée (102) conçus pour se déployer contre une paroi tissulaire ; et
une partie greffe (108) recouvrant et disposée entre la pluralité d'éléments allongés (104) et s'étendant le long de la surface intérieure du conduit (322), l'appareil à canule d'entrée ou de sortie étant **caractérisé en ce que** le conduit comprend une partie canule réglable (1100) comportant une surface filetée (1104) et une butée (1102), et la butée est conçue pour se déplacer le long d'une longueur de la surface filetée (1104) de manière à ajuster une position de la partie entrée (102) par rapport à la paroi tissulaire.

2. Appareil selon la revendication 1, ladite partie greffe (108) étant disposée sur une surface extérieure et une surface intérieure de ladite pluralité d'éléments allongés (104).

3. Appareil selon l'une quelconque des revendications 1 à 2, ladite partie greffe (108) étant disposée le long d'une surface intérieure de la pluralité d'éléments allongés (104) et s'étendant le long de la surface intérieure du conduit (322) jusqu'à une seconde extrémité du conduit ; et éventuellement
ladite partie greffe (108) s'étendant le long d'une surface extérieure de ladite pluralité d'éléments allongés (104).

4. Appareil selon l'une quelconque des revendications 1 à 3, ladite partie entrée (102) étant conçue pour se conformer à la paroi tissulaire et réduire l'érosion tissulaire de celle-ci.

5. Appareil selon l'une quelconque des revendications 1 à 4, ladite surface extérieure du conduit (322) étant conçue pour diminuer la formation de thrombus.

6. Appareil selon l'une quelconque des revendications 1 à 5, ladite partie entrée (102) comprenant une forme d'entonnoir dans une configuration initiale et étant conçue pour s'aplatir et se déployer contre la paroi tissulaire.

7. Appareil selon l'une quelconque des revendications 1 à 6, comprenant en outre un anneau de contrainte (650) disposé autour de la pluralité d'éléments allongés (104) de manière à maintenir la partie entrée (102) dans une configuration sensiblement cylindrique et permettre la libération de la partie entrée (102) de manière à l'aplatir et la déployer contre la paroi tissulaire ; et éventuellement
ledit anneau de contrainte (650) étant conçu pour glisser loin de la première extrémité du conduit (322) en réponse au contact avec une surface épicardique du cœur de manière à permettre la libération de la partie entrée (102).

8. Appareil selon l'une quelconque des revendications 1 à 7, comprenant en outre une pluralité de structures annulaires (324) disposées le long de la surface extérieure d'une partie écoulement de sortie du conduit (322).

9. Appareil selon l'une quelconque des revendications 1 à 8, ladite partie entrée (102) étant conçue pour se déployer contre la paroi tissulaire en réponse à la pression d'écoulement de fluide avec une chambre cardiaque.

10. Appareil selon l'une quelconque des revendications 1 à 9, ladite butée (1102) comprenant en outre un élément de verrouillage destiné à maintenir et/ou verrouiller la butée (1102) en place le long de la surface filetée (1104).
